# EUROPÄISCHE PATENTSCHRIFT

(11) **EP 2 055 324 B1**
(45) Veröffentlichungstag und Bekanntmachung des Hinweises auf die Patenterteilung: **05.09.2012**
(21) Anmeldenummer: 08016825.5
(22) Anmeldetag: 25.09.2008
(51) Int. Cl.: A61L 27/16, A61L 27/26, A61L 27/44, A61L 27/50, A61L 24/00, A61L 24/04

(54) **Initiatorsystem für selbsthärtende Kunststoffe, seine Verwendung und es enthaltende Knochenzementzusammensetzungen**
Initiator system for self-hardening plastics, its application and bone cement compounds containing the same
Système d'initiateur pour matières synthétiques autodurcissantes, son utilisation et compositions de ciment osseux en étant pourvues

(30) Priorität: 22.10.2007 DE 102007050763
(43) Veröffentlichungstag der Anmeldung: 06.05.2009
(73) Patentinhaber: Heraeus Medical GmbH, 61273 Wehrheim (DE)
(72) Erfinder: Vogt, Sebastian, Dr., 99092 Erfurt (DE); Büchner, Hubert, Dr., 64354 Reinheim (DE)
(74) Vertreter: Kühn, Hans-Christian

(56) Entgegenhaltungen:
- EP-A1- 1 872 767
- WO-A2-2007/140440
- DE-A1- 4 219 700

## Beschreibung

Die Erfindung betrifft ein Initiatorsystem für selbsthärtende Kunststoffe, seine Verwendung und es enthaltende Knochenzementzusammensetzungen.

Initiatorsysteme für die radikalische Polymerisation von Methacrylatmonomeren und anderen radikalisch polymerisierbaren Monomeren sind seit langem bekannt.

So wird in EP 0 732 098 A2 eine Kombination von Peroxiden und Metallverbindungen offenbart. Dabei kommt eine Kombination aus Cumenhydroperoxid, einer Metallverbindung und Thioharnstoff zu Anwendung. Eine ähnliche Kombination aus Thioharnstoff und einem Hydroperoxid wird in EP 1 479 364 A1 vorgeschlagen. In DE 195 01 933 A1 sind dagegen Mischungen aus Hydroperoxiden und Sikkativen offengelegt. Ein neues interessantes System basierend auf Hydroperoxiden, Acylthioharnstoffen und Kupfersalzen wird in EP 1 754 465 A1 dargestellt. Der Vorteil solcher Initiatorsysteme besteht in ihrer hohen thermischen Stabilität. Hydroperoxide sind reizende Verbindungen und daher nur bedingt geeignet zur Initiierung von PMMA-Knochenzementen, die direkten Kontakt zu vitalem Knochengewebe haben. Aus diesem Grund haben solche Initiationssysteme bisher keine breite Verwendung zur Herstellung von PMMA-Knochenzementen gefunden.

Das bei bisherigen PMMA-Knochenzementen eingesetzte Initiationssystem Dibenzoylperoxid und N,N-Dimethyl-p-toluidin hat sich prinzipiell bewährt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Das Dibenzoylperoxid liegt dabei als Feststoff im Zementpulver vor und das N,N-Dimethyl-p-toluidin ist in der Monomerkomponente gelöst. Dadurch ist das Initiationssystem lagerstabil bei Raumtemperatur. Bei der Herstellung von Zementpasten ist dieses Initiationssystem nur bedingt geeignet, weil das im Monomer gelöste Dibenzoylperoxid metastabil ist und auch schon bei Raumtemperatur in geringem Umfang spontan zerfällt. Dadurch neigen pastenförmige Zemente unter Verwendung des Initiationssystems Dibenzoylperoxid/ N,N-Dimethyl-p-toluidin und von vernetzend wirkenden Monomeren zur Spontanvernetzung und sind daher nur sehr bedingt lagerfähig.

In dentalen Anwendungen hat sich das Initiationssystem Barbitursäurederivat/Kupferlonen/Chorid-lonen prinzipiell zur Herstellung von nicht nachträglich verfärbenden Kunststoffen bewährt, wobei im Allgemeinen nur Pulver-Flüssigkeits-Systeme ausreichend lagerstabil sind. Bei Pasten sind die Barbitursäurederivate in den Monomeren der Paste gelöst. Es wurde dabei beobachtet, dass bei Verwendung von vernetzend wirkenden Monomeren infolge des Spontanzerfalls des gelösten Initiators eine Spontanvernetzung der Pasten häufig eintritt.

Zusammenfassend kann festgestellt werden, dass bisher kein hinreichend stabiles und nur gering toxisches Initiationssystem bekannt ist, das für die Herstellung von lagerstabilen, pastenförmigen PMMA-Knochenzementen geeignet ist. Bei Zweikomponenten-Knochenzementen ist nach der Vermischung der beiden Komponenten eine Verarbeitungszeit von mehreren Minuten unbedingt notwendig, damit die Totalendoprothesen korrekt positioniert werden können. Es ist bisher kein geeignetes Initiationssystem bekannt, das bei Pastenzementen, die mehrfach funktionelle Monomere enthalten, eine Verarbeitungszeit von mehreren Minuten erlaubt.

Der Erfindung liegt die Aufgabe zu Grunde, ein Initiatorsystem zu entwickeln, das für die Herstellung von lagerstabilen PMMA-Zementpasten geeignet ist und das eine sichere Initiierung der radikalischen Polymerisation von PMMA-Zementpasten ermöglicht. Die Polymerisation soll durch das Initationssystem verzögert initiiert werden, damit eine Verarbeitungszeit der PMMA-Zementpasten von mindestens 2,5 Minuten gewährleistet werden kann.

Grundlegend für die Erfindung war die Beobachtung, das Calcium-, Magnesium- und Eisensalze von Barbitursäurederivaten und bestimmte anorganische Kupfersalze, wie basisches Kupfercarbonat und Kupfer(II)-hydroxid, in üblichen Methacrylatmonomeren unlöslich sind. Der Erfindung liegt die Idee zu Grunde, eine Kombination von in Methacrylatmonomeren unlöslichen Salzen von Barbitursäurederivaten und in Methacrylatmonomeren unlöslichen Schwermetallsalzen zu verwenden, die unmittelbar vor der gewünschten Polymerisation durch Einwirkung von in Methacrylatmonomeren löslichen Säuren in lösliche Säureformen von Barbitursäurederivaten und im Fall der Schwermetallsalze in Methacrylatmonomer lösliche Schwermetallsalze überführt werden. Die Freisetzung der löslichen Barbitursäurederivate und die Freisetzung der löslichen Schwermetallsalze erfolgt durch Diffusion der Säure zu den unlöslichen Salzen und nachfolgend diffundieren die freigesetzten löslichen Barbitursäurederivate und Schwermetallsalze aufeinander zu. Erst bei ihrem Aufeinandertreffen kommt es in Gegenwart von ChloridIonen zur Radikalbildung und damit zur Initiierung der Polymerisation. Das bedeutet, dem eigentlichen Initiierungsschritt werden Lösungs- und Diffusionsprozesse vorangestellt, die geschwindigkeitsbestimmend für die Initiierung der Polymerisation sind.

Die Aufgabe der Erfindung wurde durch ein Initiatorsystem für selbsthärtende Kunststoffe gelöst, dass die Komponenten
a) mindestens ein in Methacrylatmonomeren unlösliches Salz einer Dialkylbarbitursäure und/oder einer Alkylcycloalkylbarbitursäure und/oder Alkylarylbarbitursäure und/oder einer Cycloalkylarylbarbitursäure,
b) mindestens ein in Methacrylatmonomeren unlösliches Schwermetallsalz,
c) mindestens ein in Methacrylatmonomeren löslichen Halogenid-lonendonator und
d) mindestens eine in Methacrylatmonomeren lösliche Säure
enthält.

Unter dem Begriff Methacrylatmonomer werden in der Polymerchemie übliche Methacrylatmonomere bezeichnet. Zu diesen gehören Methylmethacrylat, Ethylmethacrylat, Propylmethacrylat, Butylmethacrylat, Hexylmethacrylat, Cyclohexylmethacrylat, Octylmethacrylat, Decylmethacrylat, Ethylenglykoldimethacrylat, Propan-1,2-diol-dimethacrylat, Propan-1,2-diol-dimethacrylat, Butan-1,4-diol-dimethacrylat, Hexan-1,6-diol-dimethacrylat, Octan-1,8-dio-dimethacrylat, Diethylenglykoldimethacrylat, Triethylenglykoldimethacrylat, Tetraethylenglykoldimethacrylat. Daneben gehören auch BisGMA und auch Methacrylat-terminierte Makromere zu den Methacrylatmonomeren.

Die in einem Methacrylatmonomeren suspendierten Komponenten a) und b) können dabei vorteilhafterweise durch Einwirkung der in Methacrylatmonomeren löslichen Säure in in Methacrylatmonomeren lösliche Dialkylbarbitursäure und/oder Alkylcycloalkylbarbitursäure und/oder Alkylarylbarbitursäure und/oder Cycloalkylarylbarbitursäure und in Methacrylatmonomeren lösliche Schwermetallsalze der Säure überführt werden.

Calciumsalze, Magnesiumsalze und Eisensalze von Dialkylbarbitursäuren, Alkylcycloalkylbarbitursäuren, Alkylarylbarbitursäuren und Cycloalkylarylbarbitursäuren sind bevorzugt.

Calciumsalze von 1-Cyloalkyl-5-alkylbarbitursäuren und 1-Phenyl-5-alkyl-barbitursäuren sind besonders bevorzugt, wobei das Calciumsalz der 1-Cyclohexyl-5-ethyl-barbitursäure ganz besonders bevorzugt ist.

Als Schwermetallsalze sind Kupfer(II)-hydroxid, basisches Kupfercarbonat, Eisen(II)-carbonat, Mangan(II)-carbonat und Cobalt(II)-carbonat bevorzugt.

Als Halogenidionen-Donator(en) sind erfindungemäß Tetraalkylammoniumchloride bevorzugt, wobei das Trioctylmethylammoniumchlorid besonders bevorzugt ist.

Als in Methacrylatmonomeren lösliche Säure kommen vorzugsweise 2-Ethylhexansäure, Hexansäure, Heptansäure, Octansäure und Malonsäure in Frage. Daneben ist es auch möglich, Monomere mit Säurefunktionen, wie Sulfonsäure-, Phosphorsäure-, Phosphonsäure- und Carbonsäuregruppen, als lösliche Säuren zu verwenden. Es ist auch möglich, Essigsäure, Propionsäure, Pivalinsäure, Chloressigsäure, Methansulfonsäure und Phosphorsäure zu verwenden. Besonders vorteilhaft ist die Verwendung von in Methacrylatmonomeren löslichen Säuren, die mit Calcium-Ionen in Wasser schwerlösliche Salze bilden.

Es wird besonders eine Kombination aus dem Calciumsalz der 1-Cyclohexyl-5-ethylbarbitur-säure, basischem Kupfer(II)-carbonat, Trioctylammoniumchlorid und 2-Ethyl-hexansäure bevorzugt - oder eine Kombination aus dem Calciumsalz der 1-Cyclohexyl-5-ethylbarbitursäure, Kupfer(II)-hydroxid, Trioctylammoniumchlorid und 2-Ethyl-hexansäure.

Vor der Vermischung der Komponenten a), b), c) und d) können die Komponenten a) und b) in einer Paste oder einem Pulver oder einer Flüssigkeit dispergiert sein und die Komponenten c) und d) separat in einer zweiten Paste oder einem Pulver oder einer Flüssigkeit.

Die Erfindung betrifft die Verwendung des oben beschriebenen Initiatorsystems zur Zubereitung von Kombinationen Paste/Paste, Paste/Pulver, Paste/Flüssigkeit, Pulver/Flüssigkeit und Flüssigkeit/Flüssigkeit zur Herstellung von medizinischen Kunststoffen und dentalen Werkstoffen.

Das erfindungsgemäße Initiatorsystem ist bevorzugt in einer Knochenzementzusammensetzung enthalten, bei der eine pastenförmige Komponente A,
zusammengesetzt aus mindestens einem Methacrylatmonomer, aus mindestens einem in Methacrylatmonomeren löslichen Polymethylmethacrylat, einem in Methacrylatmonomeren nicht löslichen Polymethylmethacrylat, einem im Methacrylatmonomer unlöslichen Salz einer Dialkylbarbitursäure und/oder einer Alkylcycloalkylbarbitursäure und/oder Alkylarylbarbitursäure und/oder einer Cycloalkylarylbarbitursäure und mindestens einem in Methacrylatmonomeren unlöslichen Schwermetallsalz,
und eine pastenförmige Komponente B, zusammengesetzt aus mindestens einem Methacrylatmonomer, aus mindestens einem in Methacrylatmonomeren löslichen Polymethylmethacrylat, einem in Methacrylatmonomeren nicht löslichen Polymethylmethacrylat, einem in Methacrylatmonomeren löslichen Halogenid-Ionendonator und mindestens einer in Methacrylatmonomeren löslichen Säure, vorhanden sind.

Unter dem Begriff Polymethylmethacrylat werden Homopolymere des Methylmethacrylates und auch Copolymere von Methylmethacrylat und anderen Monomeren, wie Methylacrylat, Ethylacrylat, Ethylmethacrylat, Propylmethacrylat, Butylacrylat, Styren und Methylstyren verstanden.

Die Erfindung wird durch nachstehende Beispiele näher erläutert, ohne jedoch die Erfindung einzuschränken. Teile- und Prozentangaben beziehen sich wie in der übrigen Beschreibung auf das Gewicht, sofern nicht anders angegeben.

### Beispiel 1

### Synthese des Calciumsalzes der 1-Cyclohexyl-5-ethyl-barbitursäure (CaCHEBA)

Es wurden in 50 ml Methanol 10,000 g (42 mmol) 1-Cyclohexyl-5-ethyl-barbitursäure und 1,621 g (21 mmol) Calciumhydroxid unter Rühren suspendiert. Anschließend wurde eine Stunde bei Raumtemperatur weitergerührt. Danach wurde das Methanol mit einem Vakuumrotationsverdampfer abgezogen und der verbleibende Rückstand ohne weitere Reinigungsoperationen im Vakuum bis zur Massekonstanz getrocknet, wobei ein farbloser Feststoff anfiel. Ausbeute: 11,000 g (97,8 %)
FT-IR ν (cm-¹): 3211; 3134; 3083; 2940; 2857; 1748; 1711; 1664; 1427; 1364; 1319; 1260; 1207; 1136; 1088; 1075; 1043; 998; 896; 858; 805; 768; 754; 736; 717; 666.

### Beispiel 2

### Herstellung eines Zirkoniumdioxid-Kupfercarbonat-Gemischs

Es wurden 20,000 g Zirkoniumdioxid-Pulver mit 40 mg basischem Kupfer(II)-carbonat (CuCO₃×Cu(OH)₂) durch intensives Mahlen vermischt.

### Beispiel 3

### Herstellung eines Zirkoniumdioxid-Kupfercarbonat-Gemischs

Es wurden 10,000 g Zirkoniumdioxid-Pulver mit 20 mg Kupfer(II)-hydroxid (stabilisiertes Cu(OH)₂) durch intensives Mahlen vermischt.

### Beispiel 4

### Herstellung einer Polvmer-Lösung 1

Es wurden bei Raumtemperatur unter intensivem Rühren in 85,0 g Hexan-1,6-diol-dimethacrylat 15,0 g eine Poly-methylmethacrylat-co-methylacrylat (Molmasse ca. 600000; ca. 50 % Methylacrylat-Anteil) gelöst. Es entstand eine viskose klare Lösung.

### Beispiel 5

### Herstellung einer Polymer-Lösung 2

Es wurden bei Raumtemperatur unter intensivem Rühren in 90,0 g Hexan-1,6-diol-dimethacrylat 10,0 g eine Poly-methylmethacrylat-co-methylacrylat (Molmasse ca. 600000; ca. 50 % Methylacrylat-Anteil) gelöst. Es bildete sich eine viskose klare Lösung.

Für die nachfolgend in den Beispielen 6-13 beschriebenen Pasten wurde ein durch Suspensionspolymerisation hergestelltes partikuläres Poly-methylmethacrylat-co-methylacrylat (Molmasse ca. 800000; ca. 50 % Methylacrylat-Anteil, Korngröße < 63 µm) verwendet, das als Polymer 1 bezeichnet wird.

### Beispiel 6

### Paste 1

Die Herstellung der Pasten A und B erfolgte durch einfaches Verkneten. Die Paste A und die Paste B stellten streichbare, visuell homogene Pasten dar, die problemlos miteinander vermischt werden konnten.

| Pasten-Komponenten | Zusammensetzung | |
|---|---|---|
| | Paste A | Paste B |
| Polymer 1 | 4,998 g | 5,250 g |
| Polymer-Lösung 1 | 3,500 g | 3,500 g |
| Zirkoniumdioxid-Kupfercarbonat-Gemisch | 1,002 g | - |
| Zirkoniumdioxid | - | 1,000 g |
| CaCHEBA | 0,500 g | |
| 2-Ethyl-hexansäure | - | 0,200 g |
| ALIQUAT 336 | - | 0,050 g |

Nach Vermischung der Komponenten A und B war die entstandene Paste problemlos formbar und streichbar. Die Aushärtung setzte nach 2 Minuten und 50 Sekunden nach dem Vermischen ein.

### Beispiel 7

### Paste 2

| Pasten-Komponenten | Zusammensetzung | |
|---|---|---|
| | Paste A | Paste B |
| Polymer 1 | 4,998 g | 5,250 g |
| Polymer-Lösung 1 | 3,500 g | 3,500 g |
| Zirkoniumdioxid-Kupfercarbonat-Gemisch | 0,501 g | - |
| Zirkoniumdioxid | 0,501 g | 1,000 g |
| CaCHEBA | 0,500 g | |
| 2-Ethyl-hexansäure | - | 0,200 g |
| ALIQUAT 336 | - | 0,050 g |

Die Aushärtung setzte 4 Minuten und 10 Sekunden nach dem Vermischen der Komponenten A und B ein.

### Beispiel 8

### Paste 3

| Pasten-Komponenten | Zusammensetzung | |
|---|---|---|
| | Paste A | Paste B |
| Polymer 1 | 4,998 g | 5,250 g |
| Polymer-Lösung 1 | 3,500 g | 3,500 g |
| Zirkoniumdioxid-Kupfercarbonat-Gemisch | 0,250 g | - |
| Zirkoniumdioxid | 0,752 g | 1,000 g |
| CaCHEBA | 0,500 g | |
| 2-Ethyl-hexansäure | - | 0,200 g |
| ALIQUAT 336 | - | 0,050 g |

Die Aushärtung setzte 6 Minuten und 15 Sekunden nach dem Vermischen ein.

### Beispiel 9

### Paste 4

| Pasten-Komponenten | Zusammensetzung | |
|---|---|---|
| | Paste A | Paste B |
| Polymer 1 | 4,998 g | 5,250 g |
| Polymer-Lösung 1 | 3,500 g | 3,500 g |
| Zirkoniumdioxid-Kupfercarbonat-Gemisch | 1,002 g | - |
| Zirkoniumdioxid | - | 1,000 g |
| CaCHEBA | 0,500 g | |
| Octansäure | - | 0,200 g |
| ALIQUAT 336 | - | 0,050 g |

Nach Vermischung der Komponenten A und B war die Paste ebenfalls problemlos formbar und streichbar. Die Aushärtung setzte 3 Minuten und 5 Sekunden nach dem Vermischen ein.

### Beispiel 10

### Paste 5

| Pasten-Komponenten | Zusammensetzung | |
|---|---|---|
| | Paste A | Paste B |
| Polymer 1 | 4,998 g | 5,250 g |
| Polymer-Lösung 1 | 3,500 g | 3,500 g |
| Zirkoniumdioxid-Kupfercarbonat-Gemisch | 1,002 g | - |
| Zirkoniumdioxid | - | 1,000 g |
| CaCHEBA | 0,500 g | |
| Heptansäure | - | 0,200 g |
| ALIQUAT 336 | - | 0,050 g |

Nach Vermischung der Komponenten A und B war die Paste ebenfalls problemlos formbar und streichbar. Die Aushärtung setzte 3 Minuten und 5 Sekunden nach dem Vermischen ein.

### Beispiel 11

### Paste 6

| Pasten-Komponenten | Zusammensetzung | |
|---|---|---|
| | Paste A | Paste B |
| Polymer 1 | 4,998 g | 5,250 g |
| Polymer-Lösung 1 | 3,500 g | 3,500 g |
| Zirkoniumdioxid-Kupferhydroxid-Gemisch | 0,501 g | - |
| Zirkoniumdioxid | 0,501 g | 1,000 g |
| CaCHEBA | 0,500 g | |
| Heptansäure | - | 0,200 g |
| ALIQUAT 336 | - | 0,050 g |

Nach Vermischung der Komponenten A und B war die Paste ebenfalls problemlos formbar und streichbar. Die Aushärtung setzte 3 Minuten und 20 Sekunden nach dem Vermischen ein.

### Beispiel 12

### Paste 7

| Pasten-Komponenten | Zusammensetzung | |
|---|---|---|
| | Paste A | Paste B |
| Polymer | 4,998 g | 5,250 g |
| Polymer-Lösung 2 | 3,500 g | 3,500 g |
| Zirkoniumdioxid-Kupferhydroxid-Gemisch | 0,501 g | - |
| Zirkoniumdioxid | 0,501 g | 1,000 g |
| CaCHEBA | 0,500 g | |
| 2-Ethyl-hexansäure | - | 0,200 g |
| ALIQUAT 336 | - | 0,050 g |

Nach Vermischung der Komponenten A und B war die Paste ebenfalls problemlos formbar und streichbar. Die Aushärtung setzte 4 Minuten und 25 Sekunden nach dem Vermischen ein.

### Beispiel 13

### Pulver-Flüssigkeit-Zement

Es wurden 1,50 g CaCHEBA, 6 mg basisches Kupfer(II)-carbonat, 6,00 g Zirkoniumdioxid, 6,00 g Poly-methylmethacrylat-co-methylacrylat (Molmasse 600000, ca. 50 % Methylacrylat), 26,50 g Poly-methylmethacrylat-co-methylacrylat (Molmasse ca. 800000; ca. 5-8 % Methylacrylat) intensiv miteinander vermahlen. Die Monomerflüssigkeit wurde durch Vermischung von 20 ml Methylmethacrylat (stabilisiert mit 200 ppm Hydrochinon) mit 100 mg ALIQUAT 336 und 400 mg 2-Ethyl-hexansäure hergestellt. Durch Vermischung des Zementpulvers mit der Monomerflüssigkeit bildete sich ein Zementteig der ca. 8 Minuten verarbeitbar war und danach über einen Zeitraum von ca. 5 Minuten aushärtete.

## Patentansprüche

1. Initiatorsystem für selbsthärtende Kunststoffe enthaltend
a) mindestens ein in Methacrylatmonomeren unlösliches Salz einer Dialkylbarbitursäure und/oder einer Alkylcycloalkylbarbitursäure und/oder Alkylarylbarbitursäure und/oder einer Cycloalkylarylbarbitursäure,
b) mindestens ein in Methacrylatmonomeren unlösliches Schwermetallsalz,
c) mindestens einen in Methacrylatmonomeren löslichen Halogenid-lonendonator,
d) mindestens eine in Methacrylatmonomeren lösliche Säure.

2. Initiatorsystem für selbsthärtende Kunststoffe nach Anspruch 1, **dadurch gekennzeichnet, dass** die in einem Methacrylatmonomeren suspendierten Komponenten a) und b) durch Einwirkung der in Methacrylatmonomeren löslichen Säure in Methacrylatmonomeren lösliche Dialkylbarbitursäure und/oder Alkylcycloalkylbarbitursäure und/oder Alkylarylbarbitursäure und/oder Cycloalkylarylbarbitursäure und in Methacrylatmonomeren lösliche Schwermetallsalze der Säure überführbar sind.

3. Initiatorsystem für selbsthärtende Kunststoffe nach den Ansprüchen 1 und 2, **dadurch gekennzeichnet, dass** als Komponente a) Verbindungen der Gruppe bestehend aus Calciumsalzen, Magnesiumsalzen und Eisensalzen von Dialkylbarbitursäuren, Alkylcycloalkylbarbitursäuren, Alkylarylbarbitursäuren und Cycloalkylarylbarbitursäuren eingesetzt werden.

4. Initiatorsystem für selbsthärtende Kunststoffe nach einem der Ansprüche 1 bis 3, **dadurch gekennzeichnet, dass** Komponente a) der Gruppe der Calciumsalze von 1-Cyloalkyl-5-alkylbarbitursäuren und 1-Phenyl-5-alkyl-barbitursäuren entstammt.

5. Initiatorsystem für selbsthärtende Kunststoffe nach einem der Ansprüche 1 bis 4, **dadurch gekennzeichnet, dass** Komponente a) das Calciumsalz der 1-Cyclohexyl-5-ethylbarbitursäure ist.

6. Initiatorsystem für selbsthärtende Kunststoffe nach einem der Ansprüche 1 bis 5, **dadurch gekennzeichnet, dass** Komponente b) der Gruppe bestehend Kupfer(II)-hydroxid, basischem Kupfercarbonat, Eisen(II)-carbonat, Mangan(II)-carbonat und Cobalt(II)-carbonat entstammt.

7. Initiatorsystem für selbsthärtende Kunststoffe nach einem der Ansprüche 1 bis 5, **dadurch gekennzeichnet, dass** als Halogenidionen-Donator c) Tetraalkylammoniumchloride wie Trioctylmethylammoniumchlorid eingesetzt werden.

8. Initiatorsystem für selbsthärtende Kunststoffe nach einem der Ansprüche 1 und 6, **dadurch gekennzeichnet, dass** die in Methacrylatmonomeren lösliche Säure d) ein Mtiglieid der Gruppe bestehend aus 2-Ethylhexansäure, Hexansäure, Heptansäure, Octansäure oder Malonsäure ist.

9. Initiatorsystem für selbsthärtende Kunststoffe nach Anspruch 1 enthaltend a) ein Calciumsalz der 1-Cyclohexyl-5-ethylbarbitursäure, b) basisches Kupfercarbonat, c) Trioctylammoniumchlorid und d) 2-Ethyl-hexansäure.

10. Initiatorsystem für selbsthärtende Kunststoffe nach Anspruch 1 enthaltend a) das Calciumsalz der 1-Cyclohexyl-5-ethylbarbitursäure, b) Kupferhydroxid, c) Trioctylammoniumchlorid und d) 2-Ethyl-hexansäure.

11. Initiatorsystem für selbsthärtende Kunststoffe nach einem der Ansprüche 1 bis 10, **dadurch gekennzeichnet, dass** vor der Vermischung der Komponenten a), b), c) und d) die Komponenten a) und b) in einer Paste oder einem Pulver oder einer Flüssigkeit dispergiert sind und dass die Komponenten c) und d) separat in einer zweiten Paste oder einem Pulver oder einer Flüssigkeit dispergiert sind.

12. Verwendung des Initiatorsystems nach einem der Ansprüche 1-11 bei der Zubereitung von Kombinationen Paste/Paste, Paste/Pulver, Paste/Flüssigkeit, Pulver/Flüssigkeit und Flüssigkeit/Flüssigkeit zur Herstellung von medizinischen Kunstoffen und dentalen Werkstoffen.

13. Knochenzementzusammensetzung mit einer pastenförmige Komponente A, zusammengesetzt aus mindestens einem Methacrylatmonomer, aus mindestens einem in Methacrylatmonomeren löslichen Polymethylmethacrylat, einem in Methacrylatmonomeren nicht löslichen Polymethylmethacrylat, einem im Methacrylatmonomer unlöslichen Salz einer Dialkylbarbitursäure und/oder einer Alkylcycloalkylbarbitursäure und/oder Alkylarylbarbitursäure und/oder einer Cycloalkylarylbarbitursäure und mindestens einem in Methacrylatmonomeren unlöslichen Schwermetallsalz,
und einer pastenförmigen Komponente B,
zusammengesetzt aus mindestens einem Methacrylatmonomer, aus mindestens einem in Methacrylatmonomeren löslichen Polymethylmethacrylat, einem in Methacrylatmonomeren nicht löslichen Polymethylmethacrylat, einem in Methacrylatmonomeren löslichen Haloge-nid-lonendonator und mindestens einer in Methacrylatmonomeren löslichen Säure,
die ein Initiatorsystem nach einem der Ansprüche 1-11 aufweist.

## Claims

1. Initiator system for self-curing plastic materials containing
a) at least one methacrylate monomers-insoluble salt of a dialkylbarbituric acid and/or an alkylcycloalkylbarbituric acid and/or alkylarylbarbituric acid and/or cycloalkylarylbarbituric acid;
b) at least one methacrylate monomers-insoluble heavy metal salt;
c) at least one methacrylate monomers-soluble halide ion donor;
d) at least one methacrylate monomers-soluble acid.

2. Initiator system for self-curing plastic materials according to claim 1, **characterised in that** components a) and b), which are suspended in a methacrylate monomer, can be converted through the action of the methacrylate monomer-soluble acid into methacrylate monomers-soluble dialkylbarbituric acid and/or an alkylcycloalkylbarbituric acid and/or alkylarylbarbituric acid and/or cycloalkylarylbarbituric acid and methacrylate monomers-soluble heavy metal salts of the acid.

3. Initiator system for self-curing plastic materials according to claims 1 and 2, **characterised in that** compounds from the group consisting of calcium salts, magnesium salts, and iron salts of dialkylbarbituric acids, alkylcycloalkylbarbituric acids, alkylarylbarbituric acids, and cycloalkylarylbarbituric acids are used as component a).

4. Initiator system for self-curing plastic materials according to any one of the claims 1 to 3, **characterised in that** component a) is from the group of the calcium salts of 1-cycloalkyl-5-alkylbarbituric acids and 1-phenyl-5-alkylbarbituric acids.

5. Initiator system for self-curing plastic materials according to any one of the claims 1 to 4, **characterised in that** component a) is the calcium salt of 1 cyclohexyl-5-ethylbarbituric acid.

6. Initiator system for self-curing plastic materials according to any one of the claims 1 to 5, **characterised in that** component b) is from the group consisting of copper(II) hydroxide, basic copper carbonate, iron(II) carbonate, manganese(II) carbonate, and cobalt(II) carbonate.

7. Initiator system for self-curing plastic materials according to any one of the claims 1 to 5, **characterised in that** tetraalkylammoniumchlorides such as trioctylmethylammonium-chloride are used as halide ion donors.

8. Initiator system for self-curing plastic materials according to any one of the claims 1 to 6, **characterised in that** the methacrylate monomers-soluble acid is a member of the group consisting of 2-ethylhexanoic acid, hexanoic acid, heptanoic acid, octanoic acid or malonic acid.

9. Initiator system for self-curing plastic materials according to claim 1, containing a) a calcium salt of 1-cyclohexyl-5-ethylbarbituric acid, b) basic copper carbonate, c) trioctylmethylammoniumchloride, and d) 2-ethyl-hexanoic acid.

10. Initiator system for self-curing plastic materials according to claim 1, containing a) the calcium salt of 1-cyclohexyl-5-ethylbarbituric acid, b) copper hydroxide, c) trioctylmethylammoniumchloride, and d) 2-ethyl-hexanoic acid.

11. Initiator system for self-curing plastic materials according to any one of the claims 1 to 10, **characterised in that**, prior to mixing components a), b), c), components a) and b) are dispersed in a paste or a powder or a liquid and **in that** components c) and d) are separately dispersed in a second paste or a powder or a liquid.

12. Use of the initiator system according to any one of the claims 1-11 in the preparation of combinations of paste/paste, paste/powder, paste/liquid, powder/liquid, and liquid/liquid for the production of medical plastic materials and dental materials.

13. Bone cement composition comprising a paste-shaped component A, composed of at least one methacrylate monomer, at least one methacrylate monomers-soluble polymethylmethacrylate, a methacrylate monomers-insoluble polymethylmethacrylate, a methacrylate monomers-insoluble salt of a dialkylbarbituric acid and/or an alkylcycloalkylbarbituric acid and/or alkylarylbarbituric acid and/or a cycloalkylarylbarbituric acid, and at least one methacrylate monomers-insoluble heavy metal salt,
and a paste-shaped component B,
composed of at least one methacrylate monomer, at least one methacrylate monomers-soluble polymethylmethacrylate, a methacrylate monomers-insoluble polymethylmethacrylate, a methacrylate monomers-soluble halide ion donor, and at least one methacrylate monomers-soluble acid that comprises an initiator system according to any one of the claims 1-11.

## Revendications

1. Système d'amorceur pour plastiques autodurcissants contenant
a) au moins un sel insoluble dans des monomères de méthacrylate d'un acide dialkylbarbiturique et/ou d'un acide alkylcycloalkylbarbiturique et/ou d'un acide alkylarylbarbiturique et/ou d'un acide cycloalkylarylbarbiturique,
b) au moins un sel de métal lourd insoluble dans des monomères de méthacrylate,
c) au moins un donneur d'ions halogénure soluble dans des monomères de méthacrylate,
d) au moins un acide soluble dans des monomères de méthacrylate.

2. Système d'amorceur pour plastiques autodurcissants selon la revendication 1, **caractérisé en ce que** les composants a) et b) mis en suspension dans un monomère de méthacrylate peuvent être transformés par action de l'acide soluble dans des monomères de méthacrylate en acide dialkylbarbiturique et/ou acide alkylcycloalkylbarbiturique et/ou acide alkylarylbarbiturique et/ou acide cycloalkylarylbarbiturique solubles dans des monomères de méthacrylate et en sels de métal lourd solubles dans des monomères de méthacrylate de l'acide.

3. Système d'amorceur pour plastiques autodurcissants selon les revendications 1 et 2, **caractérisé en ce que** des composés du groupe constitué de sels de calcium, sels de magnésium et sels de fer d'acides dialkylbarbituriques, d'acides alkylcycloalkylbarbituriques, d'acides alkylarylbarbituriques et d'acides cycloalkylarylbarbituriques sont utilisés en tant que composant a).

4. Système d'amorceur pour plastiques autodurcissants selon l'une quelconque des revendications 1 à 3, **caractérisé en ce que** le composant a) provient du groupe des sels de calcium d'acides 1-cycloalkyl-5-alkylbarbituriques et d'acides 1-phényl-5-alkylbarbituriques.

5. Système d'amorceur pour plastiques autodurcissants selon l'une quelconque des revendications 1 à 4, **caractérisé en ce que** le composant a) est le sel de calcium de l'acide 1-cyclohexyl-5-éthylbarbiturique.

6. Système d'amorceur pour plastiques autodurcissants selon l'une quelconque des revendications 1 à 5, **caractérisé en ce que** le composant b) provient du groupe constitué d'hydroxyde de cuivre(II), de carbonate de cuivre basique, de carbonate de fer(II), de carbonate de manganèse(II) et de carbonate de cobalt(II).

7. Système d'amorceur pour plastiques autodurcissants selon l'une quelconque des revendications 1 à 5, **caractérisé en ce que** des chlorures de tétraalkylammonium comme le chlorure de trioctylméthylammonium sont utilisés en tant que donneur d'ions halogénure c).

8. Système d'amorceur pour plastiques autodurcissants selon l'une quelconque des revendications 1 et 6, **caractérisé en ce que** l'acide d) soluble dans des monomères de méthacrylate est un membre du groupe constitué de l'acide 2-éthylhexanoïque, l'acide hexanoïque, l'acide heptanoïque, l'acide octanoïque ou l'acide malonique.

9. Système d'amorceur pour plastiques autodurcissants selon la revendication 1, contenant a) un sel de calcium de l'acide 1-cyclohexyl-5-éthylbarbiturique, b) du carbonate de cuivre basique, c) du chlorure de trioctylméthylammonium et d) de l'acide 2-éthylhexanoïque.

10. Système d'amorceur pour plastiques autodurcissants selon la revendication 1, contenant a) le sel de calcium de l'acide 1-cyclohexyl-5-éthylbarbiturique, b) de l'hydroxyde de cuivre, c) du chlorure de trioctylméthylammonium et d) de l'acide 2-éthylhexanoïque.

11. Système d'amorceur pour plastiques autodurcissants selon l'une quelconque des revendications 1 à 10, **caractérisé en ce qu'**avant le mélange des composants a), b), c) et d), les composants a) et b) sont dispersés en une pâte ou une poudre ou un liquide et que les composants c) et d) sont dispersés séparément en une seconde pâte ou une poudre ou un liquide.

12. Utilisation du système d'amorceur selon l'une quelconque des revendications 1 à 11 lors de la préparation de combinaisons pâte/pâte, pâte/poudre, pâte/liquide, poudre/liquide et liquide/liquide pour la fabrication de plastiques médicinaux et de matériaux dentaires.

13. Composition de ciment osseux comprenant un composant pâteux A,
composé d'au moins un monomère de méthacrylate, d'au moins un polyméthylméthacrylate soluble dans des monomères de méthacrylate, d'un polyméthylméthacrylate non soluble dans des monomères de méthacrylate, d'un sel insoluble dans le monomère de méthacrylate d'un acide dialkylbarbiturique et/ou d'un acide alkylcycloalkylbarbiturique et/ou d'un acide alkylarylbarbiturique et/ou d'un acide cycloalkylarylbarbiturique et d'au moins un sel de métal lourd insoluble dans des monomères de méthacrylate,
et un composant pâteux B,
composé d'au moins un monomère de méthacrylate, d'au moins un polyméthylméthacrylate soluble dans des monomères de méthacrylate, d'un polyméthylméthacrylate non soluble dans des monomères de méthacrylate, d'un donneur d'ions halogénure soluble dans des monomères de méthacrylate et d'au moins un acide soluble dans des monomères de méthacrylate,
qui présente un système d'amorceur selon l'une quelconque des revendications 1 à 11.
